# FASCICULE DE BREVET EUROPEEN

(11) **EP 1 567 478 B1**
(45) Date de publication et mention de la délivrance du brevet: **21.10.2009**
(21) Numéro de dépôt: 03789490.4
(22) Date de dépôt: 25.11.2003
(51) Int. Cl.: C07C 235/10

(54) **PROCEDE DE FABRICATION DE COMPOSES NITRILES A PARTIR DE COMPOSES A INSATURATION ETHYLENIQUE**
VERFAHREN ZUR HERSTELLUNG VON NITRILEN AUS ETHYLENISCH UNGESÄTTIGTEN VERBINDUNGEN
METHOD OF PRODUCING NITRILE COMPOUNDS FROM ETHYLENICALLY-UNSATURATED COMPOUNDS

(30) Priorité: 02.12.2002 FR 0215115
(43) Date de publication de la demande: 31.08.2005
(73) Titulaire: Rhodia Opérations, 93300 Aubervilliers (FR)
(72) Inventeur: BOURGEOIS, Damien, F-69003 Lyon (FR); GALLAND, Jean-Christophe, PRINCETON, NJ, 08540 (US); DIDILLON, Blaise, F-78740 Vaux sur Seine (FR); MARION, Philippe, F-69390 Vernaison (FR)
(74) Mandataire: Esson, Jean-Pierre
(86) Numéro de dépôt international: PCT/FR2003/003475
(87) Numéro de publication internationale: WO 2004/060855

(56) Documents cités:
- EP-A- 1 201 675
- WO-A-01/21580
- WO-A-95/30680
- DE-A- 10 140 083
- FR-A- 2 338 253
- SELENT D ET AL: "NEW PHOSPHORUS LIGANDS FOR THE RHODIUM-CATALYZED ISOMERIZATION/HYDROFORMYLATION OF INTERNAL OCTENES" ANGEWANDTE CHEMIE. INTERNATIONAL EDITION, VERLAG CHEMIE. WEINHEIM, DE, vol. 40, no. 9, 4 mai 2001 (2001-05-04), pages 1696-1698, XP001009251 ISSN: 0570-0833

## Description

La présente invention concerne un procédé d'hydrocyanation de composés organiques à insaturation éthylénique en composés comprenant au moins une fonction nitrile.

Elle se rapporte plus particulièrement à l'hydrocyanation de dioléfines telles que le butadiène ou d'oléfines substituées telles que des alcènesnitriles comme les pentènenitriles.

Le brevet français n° 1 599 761 décrit un procédé de préparation de nitriles par addition d'acide cyanhydrique sur des composés organiques ayant au moins une double liaison éthylénique, en présence d'un catalyseur au nickel et d'une phosphite de triaryle. Cette réaction peut être conduite en présence ou non d'un solvant.

Lorsqu'un solvant est utilisé dans ce procédé de l'art antérieur, il s'agit de préférence d'un hydrocarbure, tel que le benzène ou les xylènes ou d'un nitrile tel que l'acétonitrile.

Le catalyseur mis en oeuvre est un complexe organique de nickel, contenant des ligands tels que les phosphines, les arsines, les stibines, les phosphites, les arsénites ou les antimonites.

La présence d'un promoteur pour activer le catalyseur, tel qu'un composé du bore ou un sel métallique, généralement un acide de Lewis, est également préconisée dans ledit brevet.

Le brevet FR-A-2 338 253 a proposé de réaliser l'hydrocyanation des composés ayant au moins une insaturation éthylénique, en présence d'une solution aqueuse d'un composé d'un métal de transition , notamment le nickel, le palladium ou le fer, et d'une phosphine sulfonée.

Les phosphines sulfonées décrites dans ce brevet sont des triarylphosphines sulfonées et plus particulièrement des triphénylphosphines sulfonées.

Ce procédé permet une hydrocyanation correcte, notamment du butadiène et des pentène-nitriles, une séparation aisée de la solution catalytique par simple décantation et par conséquent évite au maximum le rejet d'effluents ou de déchets contenant les métaux utilisés comme catalyseur.
Les brevets WO95/30680 et WO01/21580 décrivent un procédé d'hydrocyanation de composés insaturés comprenant des doubles liaison non conjugués utilisant un système catalytique à base de nickel complexé avec les ligands organophosphorés comprenant deux atomes de phosphore, appelés bidentates.
Le brevet Ep1201675 et l'article publié dans le journal ANGEWANDTE CHEMIE vol. 40, n° 9, 4 mai 2001, pages 1696-1698 décrivent des composés organophosphorés utilisés dans les systèmes catalytiques pour la réaction d'hydroformylation.

Toutefois, des recherches sont conduites pour trouver de nouveaux systèmes catalytiques plus performants tant en activité catalytique qu'en stabilité.

Un des buts de la présente invention est de proposer une nouvelle famille de ligands qui permet d'obtenir avec les métaux de transition des systèmes catalytiques présentant une activité améliorée par rapport aux systèmes connus.

A cet effet, la présente invention propose un procédé d'hydrocyanation d'un composé hydrocarboné comprenant au moins une insaturation éthylénique défini ci-après par réaction en milieu liquide avec le cyanure d'hydrogène en présence d'un catalyseur comprenant un élément métallique choisi parmi les métaux de transition et un ligand organophosphoré **caractérisé en ce que** le ligand organophosphoré répond à la formule générale (I) ci-dessous : 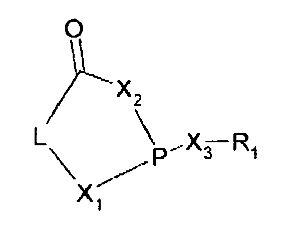 dans laquelle :
- X1, X2 identiques ou différents représentent un atome d'oxygène ou le radical divalent NR2, dans lequel R2 représente un atome d'hydrogène ou un radical alkyle, aryle, sulfonyle, cycloalkyle ou carbonylé,
- X3 représente un atome d'oxygène ou le radical divalent NR2, dans lequel R2 représente un atome d'hydrogène ou un radical alkyle, aryle, sulfonyle, cycloalkyle ou carbonylé,
- le radical R₁ représente un radical alkyle linéaire ou ramifié ayant de 1 à 12 atomes de carbone pouvant comprendre des hétéroatomes, un radical aromatique ou cycloaliphatique substitué ou non pouvant comprendre des hétéroatomes et un ou plusieurs cycles sous forme condensée ou non,
- L représente un radical divalent aromatique substitué ou non pouvant comprendre un ou plusieurs cycles sous forme condensée ou non.

Dans un mode de réalisation préféré, X1 et X2 sont différents et représentent indifféremment un atome d'oxygène ou un radical divalent NR2.

De préférence, les liaisons du radical divalent sont en position ortho dans le cas d'un radical aromatique ou portées par le même carbone ou deux carbones en position alpha l'un par rapport à l'autre dans le cas d'un radical alkyle acyclique ou cyclique.

Par ailleurs, X3 représente l'oxygène dans un mode de réalisation préféré de l'invention. En outre, L représente avantageusement :
* un dérivé de la salicylamide, éventuellement fonctionnalisé au niveau du noyau aromatique, et/ou par formation de l'amide secondaire aliphatique ou aromatique, comme par exemple la salicylanilide ou le napthol AS,
* un dérivé de l'acide anthranilique, substitué au niveau du noyau aromatique, et/ou par formation de l'amine secondaire aliphatique ou aromatique, comme par exemple l'acide N-phényl anthranilique ou l'acide N-méthyl anthranilique,
* un acide alpha aminé, plus préférentiellement un dérivé d'acide aminé naturel où l'atome d'azote est monosubstitué par un groupement aliphatique, aromatique, arylsulfonyle ou carbonylé.

Ces composés peuvent être obtenus par action, en présence d'une base, d'un composé A de formule (II) suivante :

R₁X₃H

avec un dérivé halogénophosphoré, plus particulièrement, chlorophosphoré approprié de formule (III) suivante :

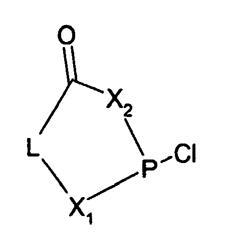

Le dérivé halogénophosphoré peut être obtenu par action de PCl₃ sur un composé de formule (IV) suivante :

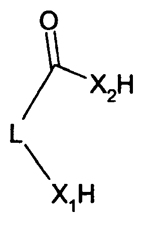

De telles structures de formule (IV) préférentielles peuvent correspondre aux composés ci-dessous :

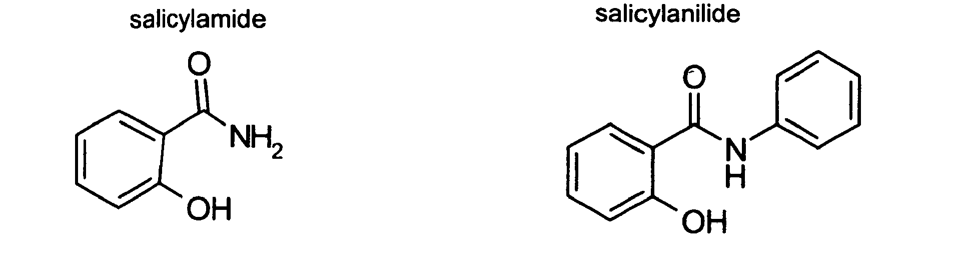

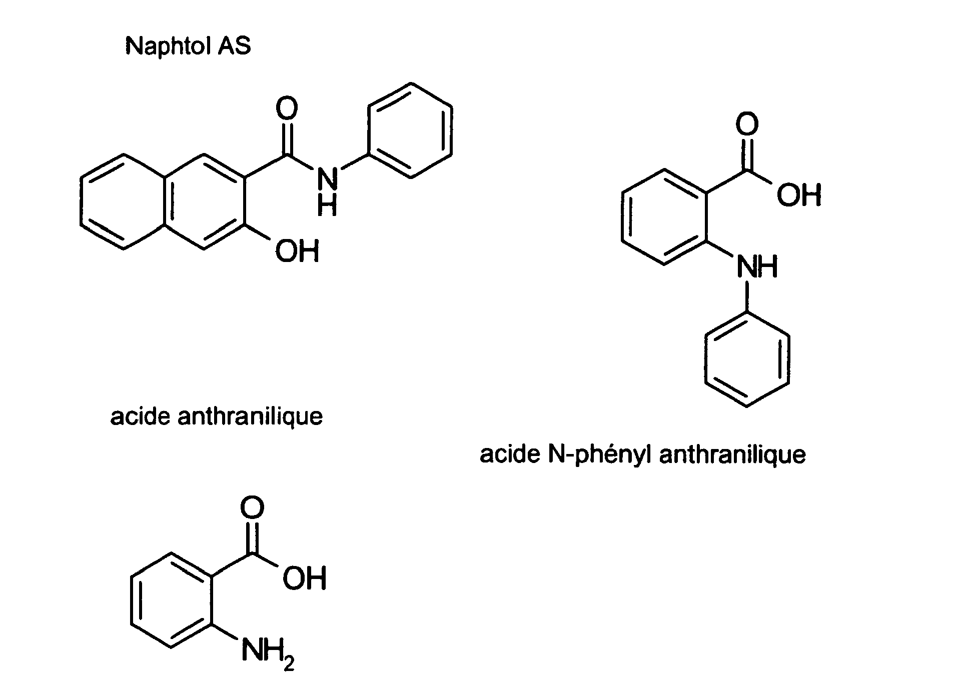

Les composés de formule (I) peuvent être obtenus par d'autres procédés notamment quand X3 représente une liaison covalente.

A titre d'exemples de composés de formule générale I, on peut citer les composés listés ci-dessous (dans les formules suivantes, le symbole Me signifie le radical méthyle, le symbole tBu le radical tertio-butyle) :

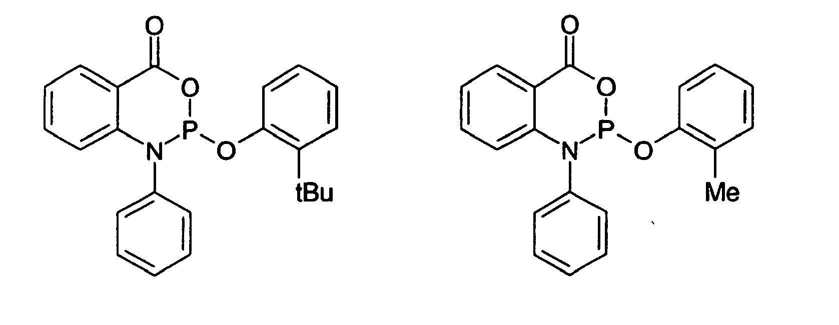

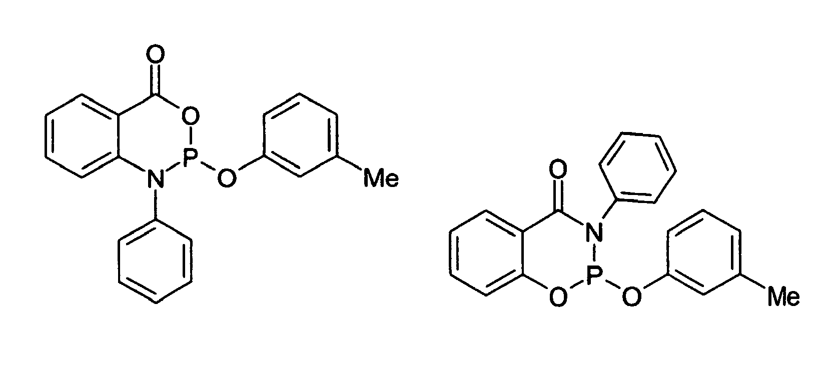

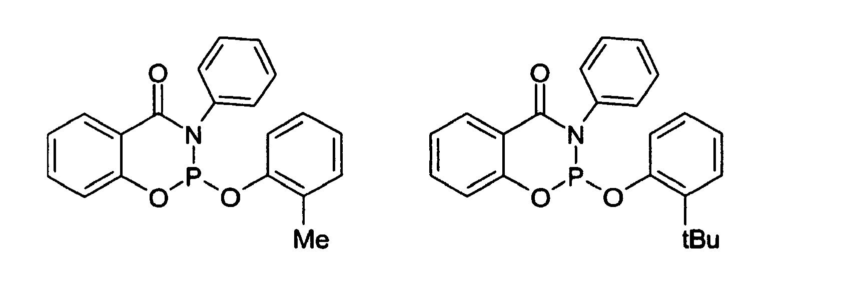

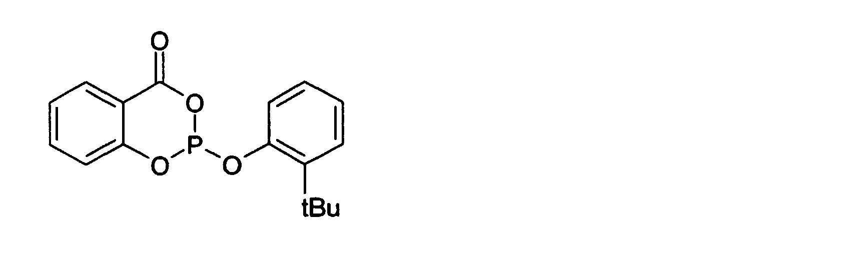

Selon une caractéristique préférée de l'invention, l'élément métallique est choisi dans le groupe comprenant le nickel, le cobalt, le fer, le ruthénium, le rhodium, le palladium, l'osmium, l'iridium, le platine, le cuivre, l'argent, l'or, le zinc, le cadmium, le mercure. Parmi ces métaux , le nickel est le métal préféré.

Selon l'invention, le catalyseur correspond, avantageusement, à la formule générale (V):

M[L_{f}]ₜ (V)

Dans laquelle:
M est un métal de transition
L_{f} représente le ligand organophosphoré de formule (I) t représente un nombre compris entre 1 et 6 (bornes incluses)

La préparation des complexes organométalliques comprenant les composés de formule (I) peut être effectuée en mettant en contact une solution d'un composé du métal choisi avec une solution d'un composé de formule (I).

Le composé du métal peut être dissout dans un solvant.

Le métal peut se trouver dans le composé mis en oeuvre, soit au degré d'oxydation qu'il aura dans le complexe organométallique, soit à un degré d'oxydation supérieur.

A titre d'exemple, on peut indiquer que dans les complexes organométalliques de l'invention, le rhodium est au degré d'oxydation (I), le ruthénium au degré d'oxydation (II), le platine au degré d'oxydation (0), le palladium au degré d'oxydation (0), l'osmium au degré d'oxydation (II), l'iridium au degré d'oxydation (I), le nickel au degré d'oxydation (0).

Si lors de la préparation du complexe organométallique, le métal est mis en oeuvre à un degré d'oxydation plus élevé, il pourra être réduit in situ.

Les complexes organométalliques comprenant les composés de formule (I) peuvent être utilisés comme catalyseurs dans les réactions d'hydrocyanation d'oléfines ou l'hydrocyanation de composés insaturés comprenant par exemple une fonction nitrile.

Comme métal de transition, les composés des métaux de transition, plus particulièrement les composés du nickel, du palladium du fer ou du cuivre sont de préférence utilisés.

Parmi les composés précités, les composés les plus préférés sont ceux du nickel.

On peut citer à titre d'exemples non limitatifs :
- les composés dans lesquels le nickel est au degré d'oxydation zéro comme le tétracyanonickelate de potassium K₄ [Ni(CN)₄], le bis (acrylonitrile) nickel zéro, le bis (cyclooctadiène-1,5) nickel (appelé également Ni(cod)₂ ) et les dérivés contenant des ligands comme le tétrakis (triphényl phosphine) nickel zéro.
- les composés du nickel comme les carboxylates (notamment l'acétate), carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

Quand le composé du nickel utilisé correspond à un état d'oxydation du nickel supérieur à 0, on ajoute au milieu réactionnel un réducteur du nickel réagissant préférentiellement avec celui-ci dans les conditions de la réaction. Ce réducteur peut être organique ou minéral. On peut citer comme exemples non limitatifs les borohydrures comme le BH₄Na, le BH₄K, la poudre de Zn, le magnésium ou l'hydrogène.

Quand le composé du nickel utilisé correspond à l'état d'oxydation 0 du nickel, on peut également ajouter un réducteur du type de ceux précités, mais cet ajout n'est pas impératif.

Quand on utilise un composé du fer, les mêmes réducteurs conviennent.

Dans le cas du palladium, les réducteurs peuvent être, en outre, des éléments du milieu réactionnel (phosphine, solvant, oléfine).

Les composés organiques comportant au moins une double liaison éthylénique plus particulièrement mis en oeuvre dans le présent procédé sont les dioléfines comme le butadiène, l'isoprène, l'hexadiène-1,5, le cyclooctadiène-1,5, les nitriles aliphatiques à insaturation éthylénique, particulièrement les pentènenitriles linéaires comme le pentène-3-nitrile, le pentène-4-nitrile, les monooléfines comme le styrène, le méthyl-styrène, le vinyl-naphtalène, le cyclohexène, le méthyl-cyclohexène ainsi que les mélanges de plusieurs de ces composés.

Les pentènenitriles notamment peuvent contenir des quantités, généralement minoritaires, d'autres composés, comme le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène, provenant par exemple de la réaction antérieure d'hydrocyanation du butadiène en nitriles insaturés.

En effet, lors de l'hydrocyanation du butadiène, il se forme avec les pentènenitriles linéaires des quantités non négligeables de méthyl-2-butène-3-nitrile et de méthyl-2-butène-2-nitrile.

Le système catalytique utilisé pour l'hydrocyanation selon le procédé de l'invention peut être préparé avant son introduction dans la zone de réaction, par exemple par addition au composé de formule (I) seul ou dissout dans un solvant, la quantité appropriée de composé du métal de transition choisi et éventuellement du réducteur. II est également possible de préparer le système catalytique "in situ" par simple addition composé de formule (I) et du composé du métal de transition dans le milieu réactionnel d'hydrocyanation avant ou après l'addition du composé à hydrocyaner.

La quantité de composé du nickel ou d'un autre métal de transition utilisée est choisie pour obtenir une concentration en mole de métal de transition par mole de composés organiques à hydrocyaner ou isomériser comprise entre 10⁻⁴ et 1, et de préférence entre 0,005 et 0,5 mole de nickel ou de l'autre métal de transition mis en oeuvre.

La quantité de composé de formule (I) utilisée pour former le catalyseur est choisie de telle sorte que le nombre de moles de ce composé rapporté à 1 mole de métal de transition soit de 0,5 à 500 et de préférence de 2 à 100.

Bien que la réaction soit conduite généralement sans solvant, il peut être avantageux de rajouter un solvant organique inerte. Le solvant peut être un solvant du catalyseur qui est miscible à la phase comprenant le composé à hydrocyaner à la température d'hydrocyanation.A titre d'exemples de tels solvants, on peut citer les hydrocarbures aromatiques, aliphatiques ou cycloaliphatiques.

La réaction d'hydrocyanation est généralement réalisée à une température de 10°C à 200°C et de préférence de 30°C à 120°C. Elle peut être réalisée en milieu monophasique ou biphasique.

Le procédé de l'invention peut être mis en oeuvre de manière continue ou discontinue.

Le cyanure d'hydrogène mis en oeuvre peut être préparé à partir des cyanures métalliques, notamment le cyanure de sodium, ou des cyanhydrines, comme la cyanhydrine de l'acétone ou par tout autre procédé de synthèse connu.

Le cyanure d'hydrogène est introduit dans le réacteur sous forme gazeuse ou sous forme liquide. II peut également être préalablement dissous dans un solvant organique.

Dans le cadre d'une mise en oeuvre discontinue, on peut en pratique charger dans un réacteur, préalablement purgé à l'aide d'un gaz inerte (tel qu'azote, argon), soit une solution contenant la totalité ou une partie des divers constituants tels que le composé à hydrocyaner, les composés de formule (I), le composé de métal de transition, les éventuels réducteur et solvant, soit séparément lesdits constituants. Généralement le réacteur est alors porté à la température choisie. Le cyanure d'hydrogène est alors lui-même introduit, de préférence de manière continue et régulière.

Quand la réaction (dont on peut suivre l'évolution par dosage de prélèvements) est terminée, le mélange réactionnel est soutiré après refroidissement et les produits de la réaction sont isolés, par exemple, par distillation.

Un perfectionnement au procédé d'hydrocyanation de composés à insaturation éthylénique selon la présente invention concerne notamment l'hydrocyanation desdits composés nitriles à insaturation éthylénique, par réaction avec le cyanure d'hydrogène et consiste à utiliser un système catalytique conforme à la présente invention avec un cocatalyseur consistant en au moins un acide de Lewis.

Les composés à insaturation éthylénique qui peuvent être mis en ouvre dans ce perfectionnement sont de manière générale ceux qui ont été cités pour le procédé de base. Cependant il est plus particulièrement avantageux de l'appliquer à la réaction d'hydrocyanation en dinitriles des nitriles aliphatiques à insaturation éthylénique, notamment aux pentènenitriles linéaires comme le pentène-3-nitrile, le pentène-4-nitrile et leurs mélanges.

Ces pentènenitriles peuvent contenir des quantités, généralement minoritaires, d'autres composés, comme le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène, provenant de la réaction antérieure d'hydrocyanation du butadiène et/ou de l'isomérisation du méthyl-2-butène-3-nitrile en pentènenitriles.

L'acide de Lewis utilisé comme cocatalyseur permet notamment, dans le cas de l'hydrocyanation des nitriles aliphatiques à insaturation éthylénique, d'améliorer la linéarité des dinitriles obtenus, c'est-à-dire le pourcentage de dinitrile linéaire par rapport à la totalité des dinitriles formés, et/ou d'augmenter l'activité et la durée de vie du catalyseur.

Par acide de Lewis, on entend dans le présent texte, selon la définition usuelle, des composés accepteurs de doublets électroniques.

On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont choisis parmi les composés des éléments des groupes Ib, IIb, IIIa, IIIb, IVa, IVb, Va, Vb, VIb, VIIb et VIII de la Classification périodique des éléments. Ces composés sont le plus souvent des sels, notamment des halogénures, comme chlorures ou bromures, sulfates, sulfonates, halogenoalkylsulfonates, perhalogénoalkylsulfonates, notamment fluoroalkylsulfonates ou perfluoroalkylsulfonates, halogénoalkylacétates, perhalogénoalkylacétates, carboxylates et phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, le trifluorométhylacétate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium.

On peut également utiliser comme acide de Lewis des composés organométalliques comme le triphénylborane, l'isopropylate de titane.
On peut bien entendu mettre en oeuvre des mélanges de plusieurs acides de Lewis.

Parmi les acides de Lewis, on préfère tout particulièrement le chlorure de zinc, le bromure de zinc, le chlorure stanneux, le bromure stanneux le triphénylborane et les mélanges chlorure de zinc/chlorure stanneux.

Le cocatalyseur acide de Lewis mis en oeuvre représente généralement de 0,01 à 50 moles par mole de composé de métal de transition, plus particulièrement de composé du nickel, et de préférence de 1 à 10 mole par mole.

Comme pour la mise en oeuvre du procédé de base de l'invention, la solution catalytique utilisée pour l'hydrocyanation en présence d'acide de Lewis peut être préparée avant son introduction dans la zone de réaction, ou in-situ par exemple par addition au milieu réactionnel des différents composants du système catalytique..

II est également possible dans les conditions du procédé d'hydrocyanation de la présente invention, et notamment en opérant en présence du catalyseur décrit précédemment comportant au moins un composé de formule (I) et au moins un composé d'un métal de transition, de réaliser, en absence de cyanure d'hydrogène, l'isomérisation du méthyl-2-butène-3-nitrile en pentènenitriles, et plus généralement des nitriles insaturés ramifiés en nitriles insaturés linéaires.

Le méthyl-2-butène-3-nitrile soumis à l'isomérisation selon l'invention peut être mis en oeuvre seul ou en mélange avec d'autres composés.

Ainsi on peut engager du méthyl-2-butène-3-nitrile en mélange avec du méthyl-2-butène-2 nitrile, du pentène-4-nitrile, du pentène-3-nitrile, du pentène-2-nitrile, du butadiène, de l'adiponitrile, du méthyl-2-glutaroronitrile, de l'éthyl-2-succinonitrile ou du valéronitrile.

II est particulièrement intéressant de traiter le mélange réactionnel provenant de l'hydrocyanation du butadiène par HCN en présence d'au moins un composé de formule (I) et d'au moins un composé d'un métal de transition, plus préférentiellement d'un composé du nickel au degré d'oxydation 0, tel que défini précédemment.

Dans le cadre de cette variante préférée, le système catalytique étant déjà présent pour la réaction d'hydrocyanation du butadiène, il suffit d'arrêter toute introduction de cyanure d'hydrogène, pour laisser se produire la réaction d'isomérisation.

On peut, le cas échéant, dans cette variante faire un léger balayage du réacteur à l'aide d'un gaz inerte comme l'azote ou l'argon par exemple, afin de chasser l'acide cyanhydrique qui pourrait être encore présent.

La réaction d'isomérisation est généralement réalisée à une température de 10°C à 200°C et de préférence de 60°C à 180°C.

Dans le cas préféré d'une isomérisation suivant immédiatement la réaction d'hydrocyanation du butadiène, il sera avantageux d'opérer à la température à laquelle l'hydrocyanation a été conduite.

Comme pour le procédé d'hydrocyanation de composés à insaturation éthylénique, le système catalytique utilisé pour l'isomérisation peut être soit déjà présent dans le milieu soit préparé selon les modes de préparation déjà décrits ci-dessus.

Bien que la réaction d'isomérisation soit conduite généralement sans solvant, il peut être avantageux de rajouter un solvant organique inerte qui pourra être celui de l'extraction ultérieure.
C'est notamment le cas lorsqu'un tel solvant a été mis en oeuvre dans la réaction d'hydrocyanation du butadiène ayant servi à préparer le milieu soumis à la réaction d'isomérisation. De tels solvants peuvent être choisis parmi ceux qui ont été cités précédemment pour l'hydrocyanation.

Toutefois, la préparation de composés dinitriles par hydrocyanation d'une oléfine comme le butadiène peut être réalisée en utilisant un système catalytique conforme à l'invention pour les étapes de formation des nitriles insaturés et l'étape d'isomérisation ci-dessus, la réaction d'hydrocyanation des nitriles insaturés en dinitriles pouvant être mis en oeuvre avec un système catalytique conforme à l'invention ou tout autre système catalytique déjà connu pour cette réaction.

De même, la réaction d'hydrocyanation de l'oléfine en nitriles insaturés et l'isomérisation de ceux-ci peuvent être réalisées avec un système catalytique différent de celui de l'invention, l'étape d'hydrocyanation des nitriles insaturés en dinitriles étant mis en oeuvre avec un système catalytique conforme à l'invention.

Les exemples qui suivent illustrent l'invention.

Dans les exemples les abréviations utilisées ont les significations indiquées ci-dessous.
cod : 1,5-cyclooctadiène.
eq : équivalent.
3PN: 3-pentènenitrile.
4PN: 4-pentènenitrile.
3+4PN : 3PN + 4PN.
TT (Y) : taux de transformation du produit à hydrocyaner Y correspondant au rapport du nombre de moles transformées de Y sur le nombre de moles initiales de Y.
Linéarité (L) : rapport du nombre de moles d'adiponitrile (AdN) formées au nombre de moles de dinitriles formées (somme des moles de AdN, éthylsuccinique dinitrile(ESN) et méthylglutaronitrile (MGN)).
CPG : chromatographie phase gazeuse.
ml : millilitre.
mol : mole.
mmol : millimole.
Ph : phényle.

### Exemple 1 : Synthèse d'un ligand A de formule suivante

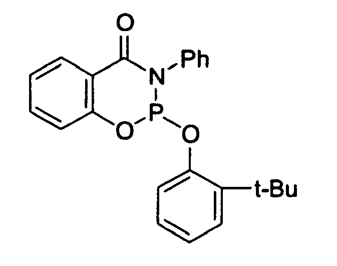

Pour la préparation du ligand **A**, on utilise comme matières premières l'ortho t-butyl phénol et une phosphochloridite dont le procédé de préparation est décrit dans un article de Sabirova, R.A. ; Nesterov, L.V. ; Arbuzov, A.E. publié dans Zh. Obshch. Khim. 1967, 37, 732-4 et de formule suivante :

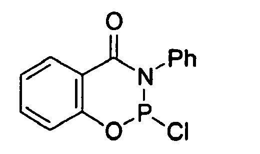

Le mode opératoire de préparation du ligand **A** est le suivant :
Sous argon, dans un ballon de 100 ml sont dissous 1.1 g de la phosphorochloridite de formule ci-dessus dans 5 ml de tétrahydrofurane anhydre et 10 ml de toluène anhydre. La solution est agitée à -10°C. Une solution de 600 mg d'ortho t-butyl phénol et de 0.85 ml de triéthylamine dans 2 ml de tétrahydrofurane anhydre est chargée dans l'ampoule de coulée et introduite goutte à goutte dans le milieu réactionnel maintenu à -10°C : un précipité blanc se forme. La suspension est agitée vigoureusement 18h à 25°C puis filtrée sous argon sur un lit d'alumine basiquel. Après rinçage au toluène, le filtrat est concentré sous pression réduite pour conduire à 1.25 g de produit brut sous forme d'une huile translucide épaisse. L'analyse RMN confirme l'identité du produit obtenu correspondant à la formule ci-dessus.

### Exemple 2 : Synthèse d'un ligand B de formule suivante

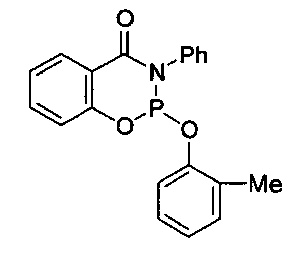

Ce ligand est préparé selon le même mode opératoire que le ligand **A**, en utilisant de l'ortho crésol : 1.67 g de la phosphochloridite et 0.65 g d'ortho crésol conduisent dans ces conditions à 1.27 g du produit correspondant à la formule ci-dessus.

### Exemple 3 : Synthèse d'un ligand C de formule suivante

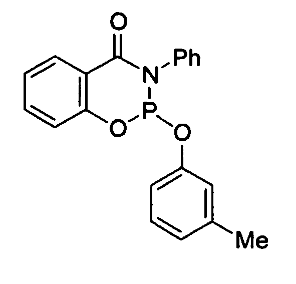

Ce ligand est préparé selon le même mode opératoire que le ligand **A**, en utilisant du meta crésol : 1.67 g de la phosphochloridite et 0.65 g de meta crésol conduisent dans ces conditions à 1.57 g du produit correspondant à la formule ci-dessus.

### Exemple 4 : Synthèse d'un ligand D de formule suivante

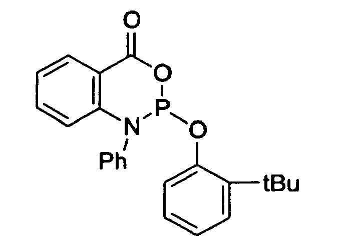

Ce ligand est obtenu par réaction de l'ortho t-butyl phénol avec une phosphorochloridite de formule suivante :

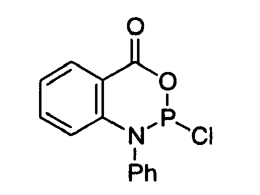

Cette phosphorochloridite est préparée selon le mode opératoire suivant :
Sous argon, dans un ballon de 100 ml, on ajoute 15 ml de PCI₃ à 10.7 g d'acide *N*-phényl anthranilique, puis 20 ml de toluène anhydre. L'épaisse suspension ainsi obtenue est agitée pendant 1h à 25°C, puis amenée progressivement à 60°C, et finalement au reflux pendant 3h. La solution ainsi obtenue est refroidie à température ambiante, filtrée sous atmosphère inerte et concentrée sous pression réduite. On obtient un solide rouge qui est purifié par trituration dans du pentane puis filtration sous atmosphère inerte. On récupère 12.5 g d'un solide rouge de pureté environ 85%.

Le mode opératoire de préparation du ligand **D** est le suivant :
Sous argon, dans un ballon de 100 ml sont dissous 1.1 g de la phosphorochloridite de formule ci-dessus dans 5 ml de tétrahydrofurane anhydre et 10 ml de toluène anhydre. La solution est agitée à -10°C. Une solution de 600 mg d'ortho t-butyl phénol et de 0.85 ml de triéthylamine dans 2 ml de tétrahydrofurane anhydre est chargée dans l'ampoule de coulée et introduite goutte à goutte dans le milieu réactionnel maintenu à -10°C : la solution initialement rouge se décolore, et un précipité jaune se forme. La suspension est agitée vigoureusement 18h à 25°C puis filtrée sous argon sur un lit d'alumine basiquel. Après rinçage au toluène, le filtrat est concentré sous pression réduite pour conduire à 1.3 g de produit brut sous forme d'une huile translucide épaisse. L'analyse RMN confirme l'identité du produit obtenu correspondant à la formule ci-dessus.

### Exemple 5 : Synthèse d'un ligand E de formule suivante

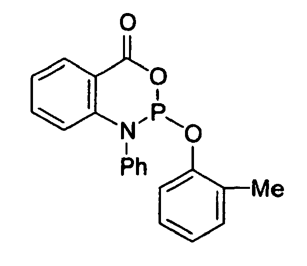

Ce ligand est préparé selon le même mode opératoire que le ligand **D**, en utilisant de l'ortho crésol : 1.67 g de la phosphochloridite et 0.65 g d'ortho crésol conduisent dans ces conditions à 1.56 g du produit correspondant à la formule ci-dessus.

### Exemple 6 : Synthèse d'un ligand F de formule suivante

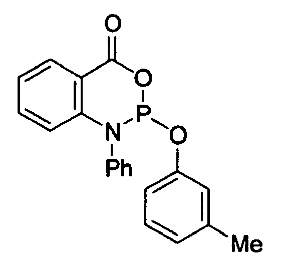

Ce ligand est préparé selon le même mode opératoire que le ligand **D**, en utilisant du meta crésol : 1.67 g de la phosphochloridite et 0.65 g de meta crésol conduisent dans ces conditions à 1.57 g du produit correspondant à la formule ci-dessus.

### Exemple 7: Hydrocyanation du 3-pentènenitrile (3PN) en adiponitrile (AdN).

Sous atmosphère d'argon, dans un tube en verre type Shott de 60 ml équipé d'un bouchon-septum, sont chargés successivement
- le ligand (2,5 eq),
- 1.21 g (15 mmol ; 30 eq) de 3PN anhydre,
- 138 mg (0,5 mmol ; 1 eq) de Ni(cod)₂ et
- 68 mg (0,5 mmol ; 1 eq) de chlorure de zinc(II).

Le mélange est porté, sous agitation, à 70°C. La cyanhydrine de l'acétone est injectée dans le milieu réactionnel par un pousse-seringue à un débit de 0,45 ml par heure. Après 3 heures d'injection, le pousse-seringue est stoppé. Le mélange est refroidi à température ambiante, dilué à l'acétone et analysé par chromatographie en phase gazeuse.

Dans ces conditions, on obtient les résultats suivants :

| *Ligand* | *TT(3PN)* | *Linéarité* |
|---|---|---|
| **A** | 36% | 44% |
| **B** | 12% | 34% |
| **C** | 24% | 74% |
| **D** | 32% | 53% |
| **E** | 10% | 72% |
| **F** | 8% | 69% |

## Revendications

1. Procédé d'hydrocyanation d'un composé hydrocarboné comprenant au moins une insaturation éthylénique choisi dans le groupe comprenant le butadiène, l'isoprène, l'hexadiène-1,5, le cyclooctadiène-1,5, le styrène, le méthyl-styrène, le vinyl-naphtalèrie, le cyclohexène, le méthyl-cyclohexène, les pentènenitriles linéaires, les mélanges de plusieurs de ces composés par réaction en milieu liquide avec le cyanure d'hydrogène en présence d'un catalyseur comprenant un élément métallique choisi parmi les métaux de transition et un ligand organophosphoré **caractérisé en ce que** le ligand organophosphoré répond à la formule générale (I) ci-dessous : 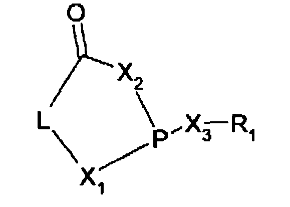 dans laquelle :
- X1, X2 identiques ou différents représentent un atome d'oxygène ou le radical divalent NR2, dans lequel R2 représente un atome d'hydrogène ou un radical alkyle, aryle, sulfonyle, cycloalkyle ou carbonylé,
- X3 représente, un atome d'oxygène ou le radical divalent NR2, dans lequel R2 représente un atome d'hydrogène ou un radical alkyle, aryle, sulfonyle, cycloalkyle ou carbonylé,
- le radical R₁ représente un radical alkyle linéaire ou ramifié ayant de 1 à 12 atomes de carbone pouvant comprendre des hétéroatomes, un radical aromatique ou cycloaliphatique substitué ou non pouvant comprendre des hétéroatomes et un ou plusieurs cycles sous forme condensée ou non,
- L représente, un radical divalent aromatique substitué ou non pouvant comprendre un ou plusieurs cycles sous forme condensée ou non.

2. Procédé selon la revendication 1, **caractérisé en ce que** X1 et X2 sont différents et représentent indifféremment un atome d'oxygène ou un radical divalent NR2.

3. Procédé selon l'une des revendications précédentes, **caractérisé en ce que** X3 représente un atome d'oxygène.

4. Procédé selon l'une des revendications précédentes, **caractérisé en ce que** L représente un radical divalent aromatique ou cyclique dont les liaisons sont en position ortho.

5. Procédé selon l'une des revendications précédentes, **caractérisé en ce que** les composés de formule générale (I) sont choisis dans le groupe comprenant les composés de formules suivantes :

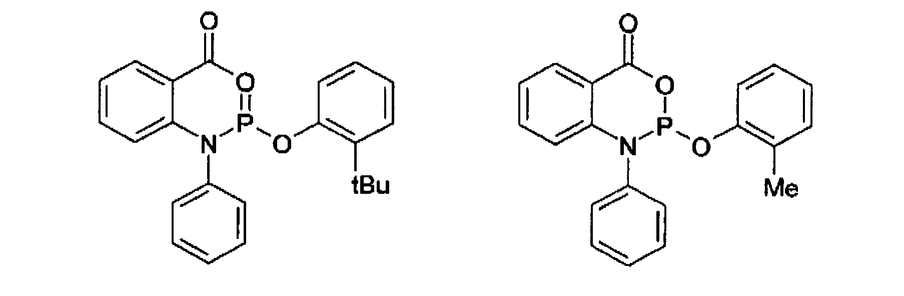

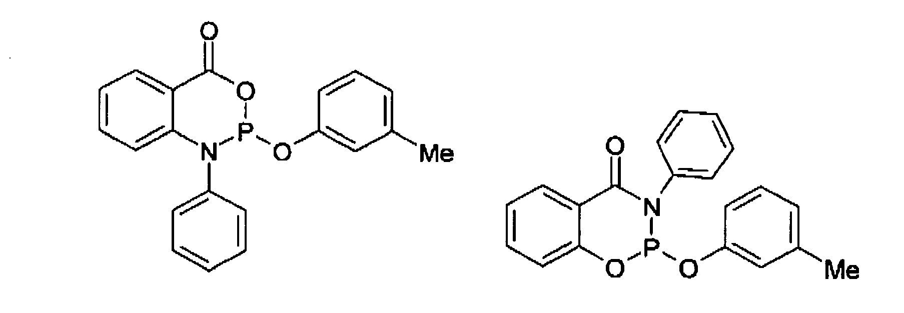

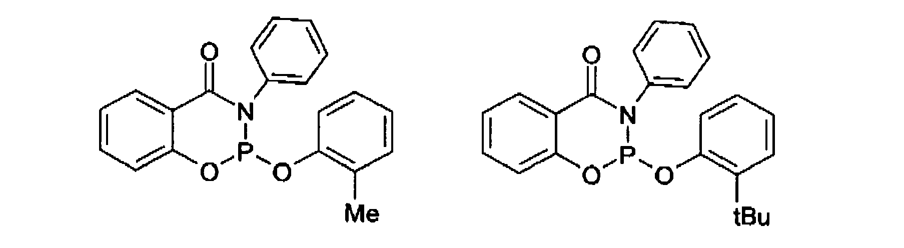

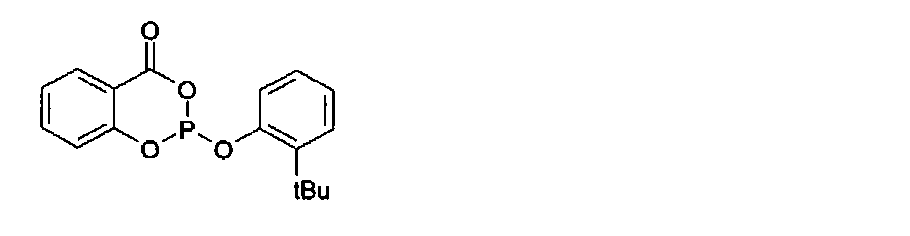

6. Procédé selon l'une des revendication précédentes, **caractérisé en ce que** l'élément métallique est choisi dans le groupe comprenant le nickel, le cobalt, le fer, le ruthénium, le rhodium, le palladium, l'osmium, l'iridium, le platine, le cuivre, l'argent, l'or, le zinc, le cadmium, le mercure.

7. Procédé selon l'une des revendications précédentes, **caractérisé en ce que** la réaction est effectuée en milieu monaphasique.

8. Procédé selon l'une des revendications précédentes **caractérisé en ce que** le catalyseur correspond à la formule générale (V):
M[L_{f}]ₜ (V)
Dans laquelle:
M est un métal de transition.
L_{f} représente le ligand organophosphoré de formule (I)
t représente un nombre compris entre 1 et 6 (bornes incluses).

9. Procédé selon l'une des revendications précédentes, **caractérisé en ce que** le milieu réactionnel comprend un solvant du catalyseur miscible à la phase comprenant le composé à hydrocyaner à la température d'hydrocyanation.

10. Procédé selon l'une des revendications précédentes, **caractérisé en ce que** les composés des métaux de transition sont ceux du nickel et sont choisis dans le groupe comprenant :
les composés dans lesquels le nickel est au degré d'oxydation zéro comme le tétracyanonickelate de potassium K₄[(Ni(CN)₄], le bis(acrylonitrile) nickel zéro, le bis(cyclooctadiène-1,5) nickel et les dérivés contenant des ligands comme le tétrakis(triphényl-phosphine) nickel zéro ;
les composés du nickel comme les carboxylates, carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfanates.

11. Procédé selon l'une des revendications précédentes, **caractérisé en ce que** la quantité de composé du nickel ou d'un autre métal de transition utilisée est choisie de telle sorte qu'il y ait par mole de composé organique à hydrocyaner ou isomériser entre 10⁻⁴ et 1 mole de nickel ou de l'autre métal de transition mis en oeuvre et **en ce que** la quantité de composés de formule (I) ou formule (II) utilisée est choisie de telle sorte que le nombre de moles de ce composé rapporté à 1 mole de métal de transition soit de 0,5 à 500.

12. Procédé selon l'une des revendications précédentes, **caractérisé en ce que** la réaction d'hydrocyanation est réalisée à une température de 10°C à 200°C.

13. Procédé selon l'une des revendications précédentes d'hydrocyanation en dinitriles de composés pentènenitriles linéaires, par réaction avec le cyanure d'hydrogène, **caractérisé en ce que** l'on opère en présence d'un système catalytique comprenant au moins un composé d'un métal de transition, au moins un composé de formule (I) et un cocatalyseur consistant en au moins un acide de Lewis.

14. Procédé selon la revendication 13, **caractérisé en ce que** les pentènenitriles linéaires sont choisis dans le gropue comprenant le pentène-3-nitrile, le pentène-4-nitrile et leurs mélanges.

15. Procédé selon la revendication 14, **caractérisé en ce que** les pentènenitriles linéaires contiennent des quantités d'autres composés choisis dans le groupe comprenant le méthyl-2-butène-3-nitrile, le méthyl-2-butène-2-nitrile, le pentène-2-nitrile, le valéronitrile, l'adiponitrile, le méthyl-2-glutaronitrile, féthyl-2-succinonitrile ou le butadiène.

16. Procédé selon l'une des revendications 13 à 15, **caractérisé en ce que** l'acide de Lewis mis en oeuvre comme cocatalyseur est choisi parmi les composés des éléments des groupes Ib, IIb, IIIa, IIIb, IVa, IVb, Va, Vb, Vlb, VIIb et VIII de la Classification périodique des éléments.

17. Procédé selon l'une des revendications 13 à 16, **caractérisé en ce que** l'acide de Lewis est choisi parmi les sels choisi dans le groupe des halogénures, sulfates, sulfonates, halogenoalkylsulfonates, perhalogénoalkylsulfonates, halogénoalkylacétates, perhalogénoalkylacétates, carboxylates et phosphates.

18. Procédé selon l'une des revendications 13 à 17, **caractérisé en ce que** l'acide de Lewis est choisi parmi le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, le trifluorométhylacétate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium et leurs mélanges, les composés organométalliques.

19. Procédé selon l'une des revendications 13 à 18, **caractérisé en ce que** l'acide de Lewis mis en oeuvre représente de 0,01 à 50 moles par mole de composé de métal de transition.

20. Procédé selon l'une des revendications 1 à 19, **caractérisé en ce que** l'on réalise en absence de cyanure d'hydrogène l'isomérisation en pentènenitriles, du méthyl-2-butène-3-nitrile présent dans le mélange réactionnel provenant de l'hydrocyanation du butadiène, en opérant en présence d'un catalyseur comportant au moins un composé de formule (I) et au moins un composé d'un métal de transition.

21. Procédé selon la revendication 20, **caractérisé en ce que** le méthyl-2-butène-3-nitrile soumis à l'isomérisation est mis en oeuvre seul ou en mélange avec du méthyl-2-butène-2-nitrile, du penténe-4-nitrile, du pentène-3-nitrile, du pentène-2-nitrile, du butadiène, de l'adiponitrile, du méthyl-2-glutaroronitrile, de l'éthyl-2-succinanitrile ou du valéronitrile.

22. Procédé selon l'une des revendications 20 ou 21, **caractérisé en ce que** la réaction d'isomérisation est réalisée à une température de 10°C à 200°C

23. Procédé selon l'une des revendications 20 à 22, **caractérisé en ce que** l'isomérisation en pentène-nitriles du méthyl-2-butène-3-nitrile est réalisée en présence d'au moins un composé d'un métal de transition, d'au moins un composé organophosphoré de formule (I) .

## Claims

1. Process for hydrocyanating a hydrocarbon-based compound containing at least one ethylenic unsaturation chosen from the group comprising butadiene, isoprene, hexa-1,5-diene, cycloocta-1,5-diene, styrene, methylstyrene, vinylnaphthalene, cyclohexene, methylcyclohexene, linear pentenenitriles and mixtures of several of these compounds, by reaction in a liquid medium with hydrogen cyanide in the presence of a catalyst comprising a metallic element chosen from transition metals and an organophosphorus ligand, **characterized in that** the organophosphorus ligand corresponds to general formula (I) below: 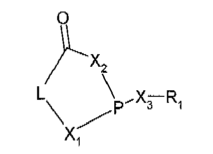 in which:
- X₁ and X₂, which may be identical or different, represent an oxygen atom or the divalent radical NR2, in which R2 represents a hydrogen atom or an alkyl, aryl, sulphonyl, cycloalkyl or carbonyl radical,
- X₃ represents an oxygen atom or the divalent radical NR2, in which R2 represents a hydrogen atom or an alkyl, aryl, sulphonyl, cycloalkyl or carbonyl radical,
- the radical R₁ represents a linear or branched alkyl radical having from 1 to 12 carbon atoms that may contain heteroatoms, or a substituted or unsubstituted aromatic or cycloaliphatic radical that may contain heteroatoms or one or more rings in fused or nonfused form,
- L represents a substituted or unsubstituted aromatic divalent radical that may contain one or more rings in fused or nonfused form.

2. Process according to Claim 1, **characterized in that** X₁ and X₂ are different and represent equally an oxygen atom or a divalent radical NR2.

3. Process according to either of the preceding claims, **characterized in that** X₃ represents an oxygen atom.

4. Process according to one of the preceding claims, **characterized in that** L represents an aromatic or cyclic divalent radical for which the bonds are in the ortho-position.

5. Process according to one of the preceding claims, **characterized in that** the compounds of general formula (I) are chosen from the group comprising the compounds of formulae below:

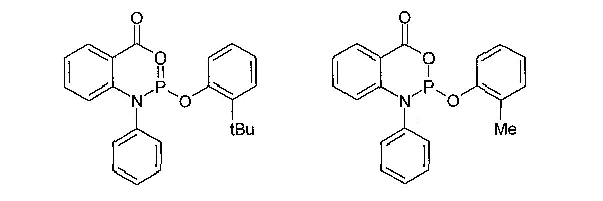

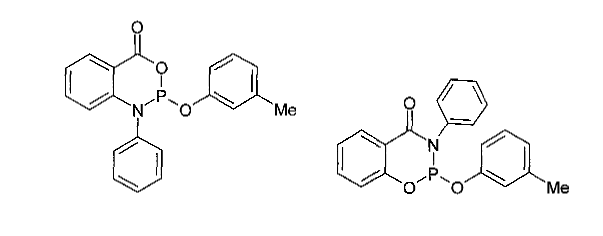

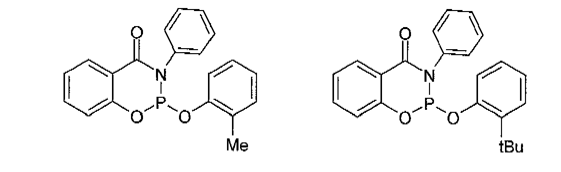

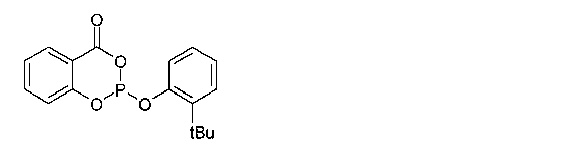

6. Process according to one of the preceding claims, **characterized in that** the metal element is chosen from the group comprising nickel, cobalt, iron, ruthenium, rhodium, palladium, osmium, iridium, platinum, copper, silver, gold, zinc, cadmium and mercury.

7. Process according to one of the preceding claims, **characterized in that** the reaction is carried out in a single-phase medium.

8. Process according to one of the preceding claims, **characterized in that** the catalyst corresponds to general formula (V):
M[L_{f}]ₜ (V)
in which:
M is a transition metal,
L_{f} represents the organophosphorus ligand of formula (I), and
t represents a number between 1 and 6 (limits inclusive).

9. Process according to one of the preceding claims, **characterized in that** the reaction medium comprises a solvent for the catalyst that is miscible with the phase comprising the compound to be hydrocyanated, at the hydrocyanation temperature.

10. Process according to one of the preceding claims, **characterized in that** the compounds of transition metals are those of nickel and are chosen from the group comprising:
- compounds in which nickel is in oxidation state zero, such as potassium tetracyanonickelate K₄[Ni(CN)₄], bis(acrylonitrile)nickel zero, bis(cycloocta-1,5-diene)nickel and derivatives containing ligands, such as tetrakis(triphenylphosphine)nickel zero;
- compounds of nickel such as carboxylates, carbonate, bicarbonate, borate, bromide, chloride, citrate, thiocyanate, cyanide, formate, hydroxide, hydrophosphite, phosphite, phosphate and derivatives, iodide, nitrate, sulphate, sulphite, aryl- and alkylsulphonates.

11. Process according to one of the preceding claims, **characterized in that** the amount of compound of nickel or of another transition metal used is chosen such that there is, per mole of organic compound to be hydrocyanated or isomerized, between 10⁻⁴ and 1 mol of nickel or of the other transition metal used, and **in that** the amount of compounds of formula (I) or formula (II) used is chosen such that the number of moles of this compound, relative to 1 mol of transition metal, is from 0.5 to 500.

12. Process according to one of the preceding claims, **characterized in that** the hydrocyanation reaction is carried out at a temperature of 10°C to 200°C.

13. Process according to one of the preceding claims, for hydrocyanating linear pentenenitrile compounds to dinitriles, by reaction with hydrogen cyanide, **characterized in that** the procedure is carried out in the presence of a catalyst system comprising at least one transition metal compound, at least one compound of formula (I) and a cocatalyst consisting of at least one Lewis acid.

14. Process according to Claim 13, **characterized in that** the linear pentenenitrile are chosen from the group comprising 3-pentenenitrile, 4-pentenenitrile, and mixtures thereof.

15. Process according to Claim 14, **characterized in that** the linear pentenenitriles contain amounts of other compounds chosen from the group comprising 2-methyl-3-butenenitrile, 2-methyl-2-butenenitrile, 2-pentenenitrile, valeronitrile, adiponitrile, 2-methylglutaronitrile, 2-ethylsuccinonitrile or butadiene.

16. Process according to one of Claims 13 to 15, **characterized in that** the Lewis acid used as cocatalyst is chosen from the compounds of elements of groups Ib, IIb, IIIa, IIIb, IVa, IVb, Va, Vb, VIb, VIIb and VIII of the Periodic Table of Elements.

17. Process according to one of Claims 13 to 16, **characterized in that** the Lewis acid is chosen from salts chosen from the group of halides, sulphates, sulphonates, haloalkylsulphonates, perhaloalkylsulphonates, haloalkylacetates, perhaloalkylacetates, carboxylates and phosphates.

18. Process according to one of Claims 13 to 17, **characterized in that** the Lewis acid is chosen from zinc chloride, zinc bromide, zinc iodide, manganese chloride, manganese bromide, cadmium chloride, cadmium bromide, stannous chloride, stannous bromide, stannous sulphate, stannous tartrate, indium trifluoromethylsulphonate, indium trifluoromethyl acetate, the chlorides or bromides of rare earth elements such as lanthanum, cerium, praseodymium, neodymium, samarium, europium, gadolinium, terbium, dysprosium, hafnium, erbium, thallium, ytterbium and lutetium, and cobalt chloride, ferrous chloride and yttrium chloride and mixtures thereof, and organometallic compounds.

19. Process according to one of Claims 13 to 18, **characterized in that** the Lewis acid used represents from 0.01 to 50 mol per mole of transition metal compound.

20. Process according to one of Claims 1 to 19, **characterized in that** the isomerization of 2-methyl-3-butenenitrile, present in the reaction mixture originating from the hydrocyanation of butadiene, to pentenenitriles is carried out, in the absence of hydrogen cyanide, by working in the presence of a catalyst comprising at least one compound of formula (I) and at least one transition metal compound.

21. Process according to Claim 20, **characterized in that** the 2-methyl-3-butenenitrile subjected to the isomerization is used alone or as a mixture with 2-methyl-2-butenenitrile, 4-pentenenitrile, 3-pentenenitrile, 2-pentenenitrile, butadiene, adiponitrile, 2-methylglutaronitrile, 2-ethylsuccinonitrile or valeronitrile.

22. Process according to either of Claims 20 and 21, **characterized in that** the isomerization reaction is carried out at a temperature of 10°C to 200°C.

23. Process according to one of Claims 20 to 22, **characterized in that** the isomerization of the 2-methyl-3-butenenitrile to pentenenitriles is carried out in the presence of at least one transition metal compound and of at least one organophosphorus compound of formula (I).

## Patentansprüche

1. Verfahren zur Hydrocyanierung einer Kohlenwasserstoffverbindung mit mindestens einer ethylenischen Ungesättigtheit aus der Gruppe bestehend aus Butadien, Isopren, 1,5-Hexadien, 1,5-Cyclooctadien, Styrol, Methylstyrol, Vinylnaphthalin, Cyclohexen, Methylcyclohexen, linearen Pentennitrilen und Mischungen mehrerer dieser Verbindungen durch Umsetzung mit Cyanwasserstoff in flüssigem Medium in Gegenwart eines Katalysators, der ein unter den Übergangsmetallen ausgewähltes Metallelement und einen Organophosphorliganden enthält, **dadurch gekennzeichnet, daß** der Organophosphorligand der nachstehenden allgemeinen Formel (I) entspricht: 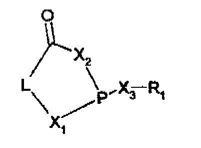 worin:
- X1 und X2 gleich oder verschieden sind und für ein Sauerstoffatom oder den zweiwertigen Rest NR2, worin R2 für ein Wasserstoffatom oder einen Alkyl-, Aryl-, Sulfonyl-, Cycloalkyl- oder Carbonylrest steht, stehen,
- X3 für ein Sauerstoffatom oder den zweiwertigen Rest NR2, worin R2 für ein Wasserstoffatom oder einen Alkyl-, Aryl-, Sulfonyl-, Cycloalkyl- oder Carbonylrest steht, steht,
- der Rest R₁ für einen linearen oder verzweigten Alkylrest mit 1 bis 12 Kohlenstoffatomen, der Heteroatome enthalten kann, einen gegebenenfalls substituierten aromatischen oder cycloaliphatischen Rest, der Heteroatome und einen oder mehrere Ringe in anellierter oder nicht anellierter Form enthalten kann, steht,
- L für einen gegebenenfalls substituierten zweiwertigen aromatischen Rest, der einen oder mehrere Ringe in anellierter oder nicht anellierter Form enthalten kann, steht.

2. Verfahren nach Anspruch 1, **dadurch gekennzeichnet, daß** X1 und X2 verschieden sind und unterschiedslos für ein Sauerstoffatom oder einen zweiwertigen Rest NR2 stehen.

3. Verfahren nach einem der vorhergehenden Ansprüche, **dadurch gekennzeichnet, daß** X3 für ein Sauerstoffatom steht.

4. Verfahren nach einem der vorhergehenden Ansprüche, **dadurch gekennzeichnet, daß** L für einen aromatischen oder cyclischen zweiwertigen Rest, dessen Bindungen sich in ortho-Position befinden, steht.

5. Verfahren nach einem der vorhergehenden Ansprüche, **dadurch gekennzeichnet, daß** man die Verbindungen der allgemeinen Formel (I) aus der Gruppe bestehend aus den Verbindungen der folgenden Formeln auswählt:

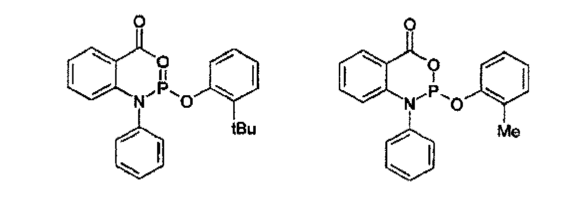

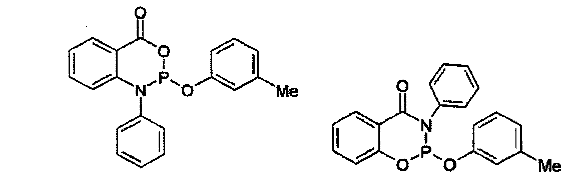

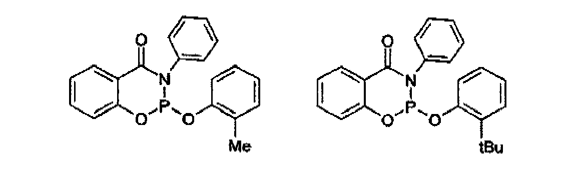

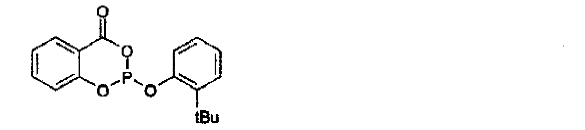

6. Verfahren nach einem der vorhergehenden Ansprüche, **dadurch gekennzeichnet, daß** man das Metallelement aus der Gruppe bestehend aus Nickel, Cobalt, Eisen, Ruthenium, Rhodium, Palladium, Osmium, Iridium, Platin, Kupfer, Silber, Gold, Zink, Cadmium und Quecksilber auswählt.

7. Verfahren nach einem der vorhergehenden Ansprüche, **dadurch gekennzeichnet, daß** man die Umsetzung in einem einphasigen Medium durchführt.

8. Verfahren nach einem der vorhergehenden Ansprüche, **dadurch gekennzeichnet, daß** der Katalysator der allgemeinen Formel (V) entspricht:
M(L_{f})ₜ (V)
worin:
M für ein Übergangsmetall steht,
L_{f} für den Organophosphorliganden der Formel (I) steht und
t für eine Zahl zwischen 1 und 6 (einschließlich der Grenzwerte) steht.

9. Verfahren nach einem der vorhergehenden Ansprüche, **dadurch gekennzeichnet, daß** das Reaktionsmedium ein Lösungsmittel für den Katalysator, das bei der Hydrocyanierungstemperatur mit der die zu hydrocyanierende Verbindung enthaltenden Phase mischbar ist, umfaßt.

10. Verfahren nach einem der vorhergehenden Ansprüche, **dadurch gekennzeichnet, daß** es sich bei den Übergangsmetallverbindungen um Nickelverbindungen handelt und man die Übergangsmetallverbindungen aus der Gruppe bestehend aus:
Verbindungen, in denen das Nickel in der Oxidationsstufe null vorliegt, wie Kaliumtetracyanonickelat K₄[Ni(CN)₄], Bis(acrylnitril)-nickel (0), Bis(1,5-cyclooctadien)nickel und Derivaten mit Liganden wie Tetrakis(triphenylphosphin)nickel(0) und
Nickelverbindungen wie Carboxylate, Carbonat, Hydrogencarbonat, Borat, Bromid, Chlorid, Citrat, Thiocyanat, Cyanid, Formiat, Hydroxid, Hydrophosphit, Phosphit, Phosphat und Derivaten, Iodid, Nitrat, Sulfat, Sulfit und Aryl- und Akylsulfonaten auswählt.

11. Verfahren nach einem der vorhergehenden Ansprüche, **dadurch gekennzeichnet, daß** man die verwendete Menge der Nickelverbindung oder eines anderen Übergangsmetalls so wählt, daß pro Mol zu hydrocyanierende oder zu isomerisierende organische Verbindung zwischen 10⁻⁴ und 1 mol Nickel oder anderes verwendetes Übergangsmetall vorliegen, und die verwendete Menge der Verbindungen der Formel (I) oder Formel (II) so wählt, daß die Zahl der Mole dieser Verbindung, bezogen auf 1 mol Übergangsmetall, 0,5 bis 500 beträgt.

12. Verfahren nach einem der vorhergehenden Ansprüche, **dadurch gekennzeichnet, daß** man die Hydrocyanierungsreaktion bei einer Temperatur von 10°C bis 200°C durchführt.

13. Verfahren nach einem der vorhergehenden Ansprüche zur Hydrocyanierung von linearen Pentennitrilen zu Dinitrilen durch Umsetzung mit Cyanwasserstoff, **dadurch gekennzeichnet, daß** man in Gegenwart eines Katalysatorsystems, das mindestens eine Übergangsmetallverbindung, mindestens eine Verbindung der Formel (I) und einen Cokatalysator, der mindestens aus einer Lewis-Säure besteht, arbeitet.

14. Verfahren nach Anspruch 13, **dadurch gekennzeichnet, daß** man die linearen Pentennitrile aus der Gruppe bestehend aus 3-Pentennitril, 4-Pentennitril und Mischungen davon auswählt.

15. Verfahren nach Anspruch 14, **dadurch gekennzeichnet, daß** die linearen Pentennitrile Mengen anderer Verbindungen aus der Gruppe bestehend aus 2-Methyl-3-butennitril, 2-Methyl-2-butennitril, 2-Pentennitril, Valeronitril, Adiponitril, 2-Methylglutarnitril, 2-Ethylsuccinnitril und Butadien enthalten.

16. Verfahren nach einem der Ansprüche 13 bis 15, **dadurch gekennzeichnet, daß** man die als Cokatalysator verwendete Lewis-Säure unter den Verbindungen der Gruppen Ib, IIb, IIIa, IIIB, IVa, IVb, Va, Vb, VIb, VIIb und VIII des Periodensystems der Elemente auswählt.

17. Verfahren nach einem der Ansprüche 13 bis 16, **dadurch gekennzeichnet, daß** man die Lewis-Säure unter Salzen aus der Gruppe der Halogenide, Sulfate, Sulfonate, Halogenalkylsulfonate, Perhalogenalkylsulfonate, Halogenalkylacetate, Perhalogenalkylacetate, Carboxylate und Phosphate auswählt.

18. Verfahren nach einem der Ansprüche 13 bis 17, **dadurch gekennzeichnet, daß** man die Lewis-Säure unter Zinkchlorid, Zinkbromid, Zinkiodid, Manganchlorid, Manganbromid, Cadmiumchlorid, Cadmiumbromid, Zinn(II)-chlorid, Zinn(II)-bromid, Zinn(II)-sulfat, Zinn(II)-tartrat, Indiumtrifluormethylsulfonat, Indiumtrifluormethylacetat, den Chloriden oder Bromiden der Seltenerdmetalle wie Lanthan, Cer, Praseodym, Neodym, Samarium, Europium, Gadolinium, Terbium, Dysprosium, Hafnium, Erbium, Thallium, Ytterbium und Lutetium, Cobaltchlorid, Eisen(II)-chlorid, Yttriumchlorid und Mischungen davon oder metallorganischen Verbindungen auswählt.

19. Verfahren nach einem der Ansprüche 13 bis 18, **dadurch gekennzeichnet, daß** die verwendete Lewis-Säure 0,01 bis 50 mol pro Mol übergangsmetallverbindung ausmacht.

20. Verfahren nach einem der Ansprüche 1 bis 19, **dadurch gekennzeichnet, daß** man das in der Reaktionsmischung vorliegende 2-Methyl-3-butennitril, das aus der Hydrocyanierung von Butadien stammt, in Abwesenheit von Cyanwasserstoff und in Gegenwart eines Katalysators, der mindestens eine Verbindung der Formel (I) und mindestens eine Übergangsmetallverbindung enthält, zu Pentennitrilen isomerisiert.

21. Verfahren nach Anspruch 20, **dadurch gekennzeichnet, daß** man das der Isomerisierung unterworfene 2-Methyl-3-butennitril alleine oder im Gemisch mit 2-Methyl-2-butennitril, 4-Pentennitril, 3-Pentennitril, 2-Pentennitril, Butadien, Adiponitril, 2-Methylglutarnitril, 2-Ethylsuccinnitril oder Valeronitril einsetzt.

22. Verfahren nach Anspruch 20 oder 21, **dadurch gekennzeichnet, daß** man die Isomerisierungsrealtion bei einer Temperatur von 10°C bis 200°C durchführt.

23. Verfahren nach einem der Ansprüche 20 bis 22, **dadurch gekennzeichnet, daß** man die Isomerisierung von 2-Methyl-3-butennitril zu Pentennitrilen in Gegenwart mindestens einer Übergangsmetallverbindung und mindestens einer Organophosphorverbindung der Formel (I) durchführt.
